# EUROPEAN PATENT APPLICATION

(11) **EP 4 817 602 A1**
(43) Date of publication of application: **07.10.2026**
(21) Application number: 26167732.2
(22) Date of filing: 25.03.2026
(51) Int. Cl.: A61K 31/00, A61K 31/519, A61P 35/00, A61P 35/02, G01N 33/573

(54) **USE OF A MEDICAMENT SIMULTANEOUSLY TARGETING THE TYROSINE KINASE ACTIVITY AND GEF ACTIVITY OF EGFR**

(30) Priority: 03.04.2025 CN 202510419394
(71) Applicant: Sun Yat-Sen University, Guangzhou, Guangdong 510275 (CN); Shanghai Institute of Organic Chemistry, Chinese Academy of Sciences, Shanghai 200032 (CN)
(72) Inventor: Kang, Tie bang, Guangdong, 510060 (CN); Ding, Ke, Shanghai, 200032 (CN); He, Xiao bo, Guangdong, 510060 (CN); Wu, Qin xue, Shanghai, 200032 (CN); Wei, Zhi hao, Guangdong, 510060 (CN)
(74) Representative: dompatent

(57) **Abstract**

The present invention provides an EGFR inhibitor for use in the preparation of a medicament for treating diseases mediated by guanosine exchange factor (GEF) activity or co-mediated by guanosine nucleotide exchange factor (GEF) activity and EGFR tyrosine kinase activity, wherein the diseases are selected from the group consisting of: lung cancer, colon cancer, breast cancer, endometrial cancer, thyroid cancer, glioma, squamous cell carcinoma, prostate cancer, melanoma, leukemia, and glioblastoma. Specifically, the present invention has discovered that in addition to its conventional tyrosine kinase activity, EGFR also possesses a GEF function that promotes the activation of Ras homologous protein (Rheb), this function is independent of its tyrosine kinase activity and relies critically on the glutamate residue at position 804 on the EGFR molecule. The Rheb-GEF activity of EGFR induces allosteric activation of mammalian target of rapamycin complex 1 (mTORC1), ultimately promoting tumor growth.

## Description

### Cross-Reference to Related Application

This patent application claims the benefit of and priority to Chines Application No. 202510419394.2, filed April 3, 2025, the content of which is incorporated herein by reference in its entirety for all purposes.

### Technical Field

The present invention relates to the field of biomedical technology, specifically to the use of a medicament that simultaneously targets the tyrosine kinase activity and GEF activity of EGFR.

### Background

Epidermal growth factor receptor (EGFR) is a transmembrane glycoprotein whose intracellular tyrosine kinase domain plays a crucial role in cellular signal transduction. Upon binding to growth factors, EGFR undergoes dimerization and autophosphorylation, thereby activating multiple downstream signaling pathways to regulate fundamental biological processes, such as cell proliferation, differentiation, survival, and migration. Under normal physiological conditions, EGFR signaling is finely regulated to maintain the balance of cellular behavior.

In various malignant tumors (such as lung cancer, colon cancer, breast cancer, endometrial cancer, thyroid cancer, glioma, squamous cell carcinoma, and prostate cancer), EGFR is often abnormally activated due to overexpression, gene amplification, or specific mutations. This abnormal activation leads to continuous EGFR signaling, resulting in sustained activation of downstream signaling pathways. For example, sustained EGFR activation indirectly and continuously stimulates the mammalian target of rapamycin complex 1 (mTORC1) signaling pathway, promoting protein synthesis and cell proliferation while inhibiting autophagy, thereby playing a key role in tumor initiation and progression.

Current EGFR inhibitors (such as Gefitinib and Osimertinib) only target the intracellular kinase domain of EGFR, leading to a high incidence of drug resistance. Studies have shown that resistant tumors may evade inhibition by activating non-kinase-dependent pathways of EGFR, however, research on these non-kinase-dependent pathways is limited, and thus fails to effectively address the issue of high EGFR resistance rates.

Therefore, there is an urgent need in this field to conduct in-depth investigations into the molecular mechanisms of EGFR-driven tumors, particularly the mechanisms by which tumor cells develop resistance through EGFR non-kinase-dependent pathways. Uncovering these underlying regulatory mechanisms will not only help explain the reasons for the limited efficacy of existing treatments, but also provide a theoretical basis and new prospects for the development of combined anti-tumor therapeutic strategies with enhanced precision, efficacy and safety.

### Summary of the Invention

To address the above issues, the present invention provides a use of a medicament that can simultaneously regulate both EGFR tyrosine kinase activity and GEF activity.

In the first aspect of the invention, provided is a use of an EGFR inhibitor for the preparation of a pharmaceutical composition for treating diseases mediated by guanine nucleotide exchange factor (GEF) activity, or co-mediated by guanine nucleotide exchange factor (GEF) activity and EGFR tyrosine kinase activity.

In another preferred embodiment, the EGFR inhibitor inhibits GEF activity.

In another preferred embodiment, the EGFR inhibitor simultaneously inhibits both EGFR tyrosine kinase activity and GEF activity.

In a preferred embodiment, the disease is a cancer selected from the group consisting of: lung cancer, colorectal cancer, breast cancer, endometrial cancer, thyroid cancer, glioma, squamous cell carcinoma, prostate cancer, melanoma, leukemia, and glioblastoma.

In a preferred embodiment, the pharmaceutical composition is further used to modulate abnormal Rheb nucleotide exchange; preferably, the regulation is independent of the regulation of EGFR kinase activity or expression abnormalities.

In another preferred embodiment, the pharmaceutical composition can simultaneously modulate EGFR kinase activity and the Rheb nucleotide exchange capacity, and the regulation of the Rheb nucleotide exchange capacity is independent of the regulation of EGFR kinase activity.

In another preferred embodiment, the pharmaceutical composition inhibits mTORC1 activation by modulating Rheb nucleotide exchange.

In another preferred embodiment, the pharmaceutical composition modulates Rheb nucleotide exchange by modulating GEF activity, and the regulation is independent of the regulation of EGFR kinase activity.

In another preferred embodiment, the pharmaceutical composition modulates GEF activity by acting on the intracellular tyrosine kinase domain of EGFR, and the regulation is independent of the regulation of EGFR kinase activity.

In another preferred embodiment, the pharmaceutical composition modulates GEF activity by acting on the glutamate residue at position 804 of EGFR, and the regulation is independent of the regulation of EGFR kinase activity.

In another preferred embodiment, the EGFR inhibitor may cause a mutation in the glutamate residue at position 804 of EGFR.

In a preferred embodiment, the pharmaceutical composition simultaneously modulates EGFR tyrosine kinase activity and GEF activity, and the regulation of GEF activity is independent of the regulation of EGFR tyrosine kinase activity.

In another preferred embodiment, the EGFR tyrosine kinase activity is caused by EGFR overexpression, EGFR gene amplification, or an EGFR-specific mutation.

In another preferred embodiment, the EGFR-specific mutation is an EGFRvIII mutant, an L858R point mutation, or an in-frame exon-19 deletion mutation.

In another preferred embodiment, the EGFR inhibitor acts simultaneously on the EGFR tyrosine kinase domain and the glutamate residue at position 804 of EGFR.

In another preferred embodiment, the action comprises: modifying gene regions associated with EGFR tyrosine kinase activity and gene regions associated with GEF activity of the glutamate residue at position 804 of EGFR.

In a preferred embodiment, the EGFR inhibitor is selected from the group consisting of: small molecule compounds, nanomaterial drugs, gene editing technologies, protein antibodies, nucleic acid drugs, enzyme drugs, expression vectors, cell therapies, hormone drugs, peptide drugs, or combinations thereof.

In another preferred embodiment, the nanomaterial drug is a liposomal drug.

In another preferred embodiment, the nucleic acid drug is selected from the group consisting of: mRNA, siRNA, shRNA, microRNA, oligonucleotides, polynucleotides, aptamers, or combinations thereof.

In another preferred embodiment, the enzyme drug is selected from the group consisting of: DNAase, ribozyme, or a combination thereof.

In another preferred embodiment, the cell therapy is CAR-T cell therapy.

In a preferred embodiment, the EGFR inhibitor is a small-molecule compound.

In a preferred embodiment, the EGFR inhibitor is a small-molecule compound represented by the following formula:

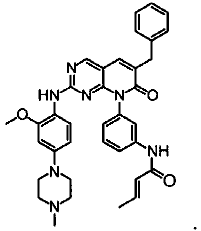

In the second aspect of the present invention, provided is a pharmaceutical combination comprising an EGFR tyrosine kinase inhibitor and a GEF activity inhibitor, or a dual-target inhibitor of EGFR tyrosine kinase and GEF activity.

In another preferred embodiment, the pharmaceutical combination may modulate the Rheb nucleotide exchange.

In the third aspect of the present invention, provided is a use of the pharmaceutical combination described in the second aspect of the present invention for the preparation of a pharmaceutical composition for treating tumors driven by EGFR or resistant to a single EGFR tyrosine kinase inhibitor;
preferably, the tumors are selected from the group consisting of: lung cancer, colorectal cancer, breast cancer, endometrial cancer, thyroid cancer, glioma, squamous cell carcinoma, prostate cancer, melanoma, leukemia, and glioblastoma.

In the fourth aspect of the present invention, provided is a method for designing a dual-target inhibitor of EGFR tyrosine kinase and GEF activity, comprising the steps of:
(a) providing a candidate molecule, wherein the candidate molecule can simultaneously act on the kinase domain of EGFR and the glutamate residue at position 804 of EGFR;
(b) determining the inhibitory ability of the candidate molecule against EGFR kinase and GEF activity.

In the fifth aspect of the present invention, provided is a method for screening compounds for the treatment of diseases mediated by GEF activity, or co-mediated by GEF activity and EGFR tyrosine kinase activity, comprising the following steps:
(A) administering the candidate drug to an animal model of a disease associated with EGFR tyrosine kinase activity and GEF activity;
(B) detecting the Rheb nucleotide exchange capacity in the animal model, wherein a drug that reduces the Rheb nucleotide exchange capacity is selected as a target drug.

In the sixth aspect of the present invention, provided is a method for treating diseases mediated by GEF activity, or co- mediated by GEF activity and EGFR tyrosine kinase activity, wherein the method comprises the step of: administering a pharmaceutical combination comprising an EGFR tyrosine kinase inhibitor and a GEF activity inhibitor, or a dual-target inhibitor of EGFR tyrosine kinase/GEF activity, to a subject in need thereof.

The subject is selected from the group consisting of: humans, mice, rats, guinea pigs, pigs, dogs, monkeys, and apes.

It should be understood that, within the scope of the present invention, the aforementioned technical features of the present invention and the technical features specifically described below (e.g., in the examples) may be combined with each other to form a new or preferred technical solution. Due to space limitations, it will not be listed one by one here.

### Brief Description of the Drawings

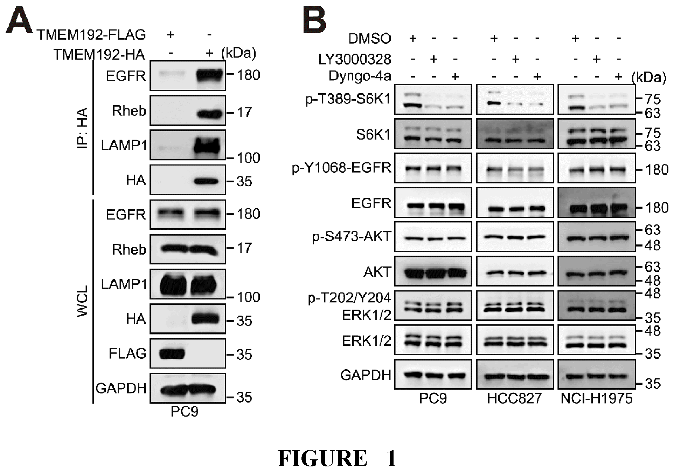

Figure 1 shows that preventing activated EGFR from localizing to the lysosomal surface can significantly inhibit mTORC1 activation. In Figure 1, A: Activated EGFR is able to localize to the lysosomal surface; B: Following treatment with an endocytosis inhibitor or a cathepsin S inhibitor (an inhibitor of late endosomal-lysosomal trafficking), mTORC1 activation is significantly inhibited, while EGFR kinase activity remains unaffected.

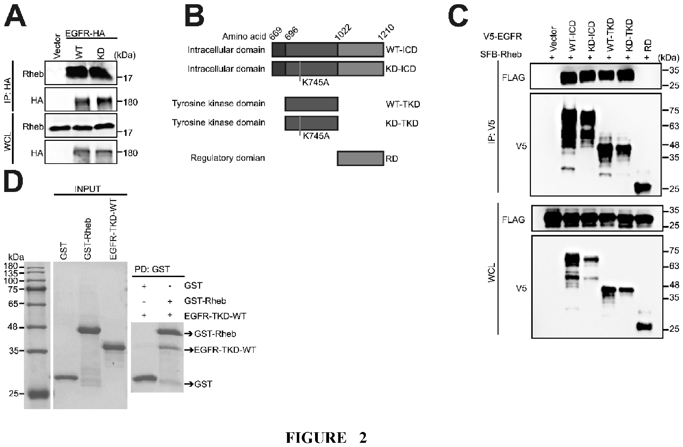

Figure 2 shows a direct interaction between EGFR and Rheb that is independent of EGFR kinase activity. In Figure 2, A: The interaction between EGFR and Rheb is independent of EGFR kinase activity; B: Schematic representation of different intracellular truncated variants of EGFR; C: EGFR primarily interacts with Rheb through its intracellular tyrosine kinase domain; D: The intracellular tyrosine kinase domain of EGFR can bind directly to Rheb.

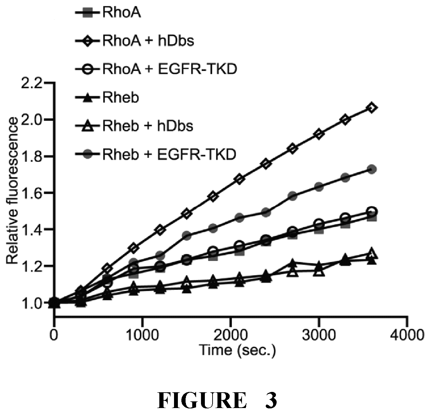

Figure 3 shows that EGFR-TKD promotes Rheb nucleotide exchange.

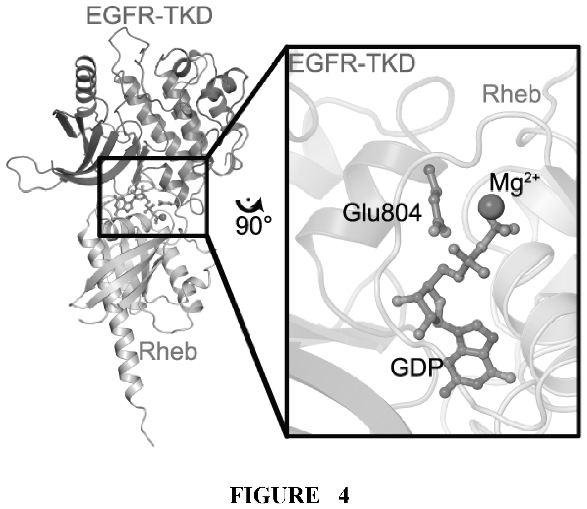

Figure 4 shows a schematic diagram of the structural model obtained by predicting the binding of the EGFR-Rheb complex based on AlphaFold2.

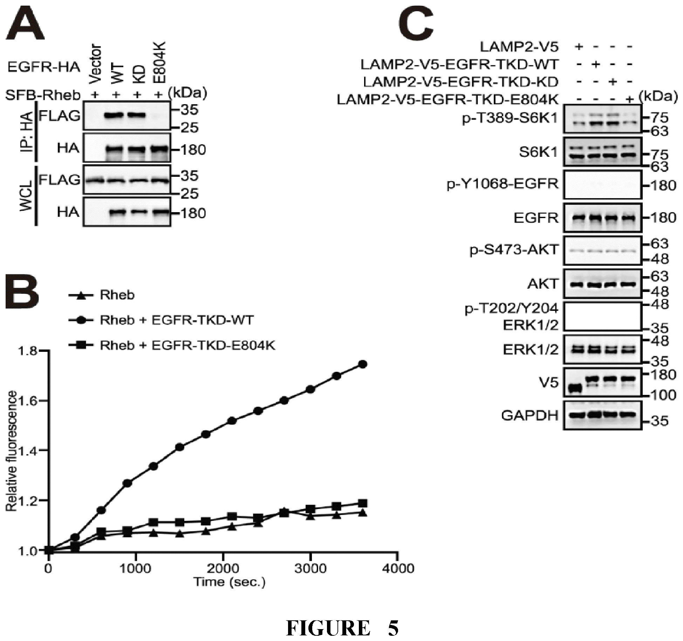

Figure 5 shows the effect of the glutamate residue at position 804 of EGFR on its GEF activity. In Figure 5: A: The EGFR-E804K mutant loses its ability to interact with Rheb; B: The EGFR-TKD-E804K mutant fails to promote Rheb nucleotide exchange; C: The lysosome-localized LAMP2-V5-EGFR-TKD-E804K fusion protein fails to activate mTORC1, whereas both wild-type (WT) and kinase-deficient (KD) EGFR-TKD effectively activate mTORC1.

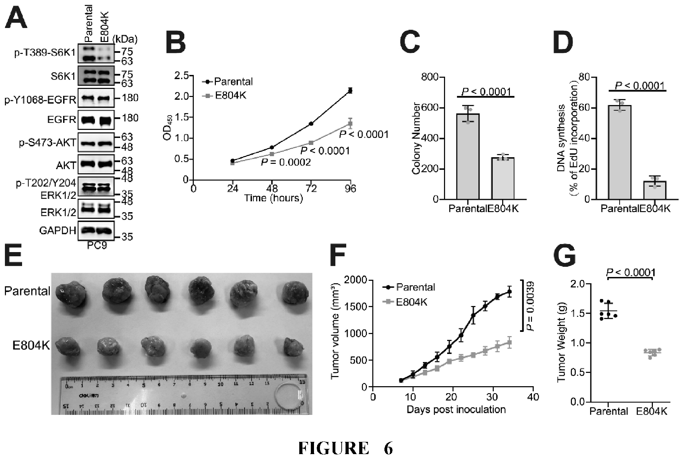

Figure 6 shows the effects of CRISPR-Cas9-mediated Knock-in of the EGFR-E804K mutation in PC9 lung cancer cells on the mTORC1 signaling pathway and cell proliferation. In Figure 6: A: PC9 cells with the EGFR-E804K knock-in were unable to activate mTORC1, but their EGFR kinase activity remained unaffected; B: The proliferative capacity of parental PC9 cells and EGFR-E804K knock-in cells was evaluated by CCK-8 assay; C: The clonogenic capacity of parental PC9 cells and EGFR-E804K knock-in cells was evaluated by clonogenic assay; D: DNA synthesis capacity in parental PC9 cells and EGFR-E804K knock-in cells was detected by EdU assay; E: Schematic diagram of subcutaneous tumor implantation of parental PC9 cells and EGFR-E804K knock-in cells in mice; F: Volume changes of the subcutaneous tumors shown in Figure E; G: Weight changes of the subcutaneous tumors shown in Figure E.

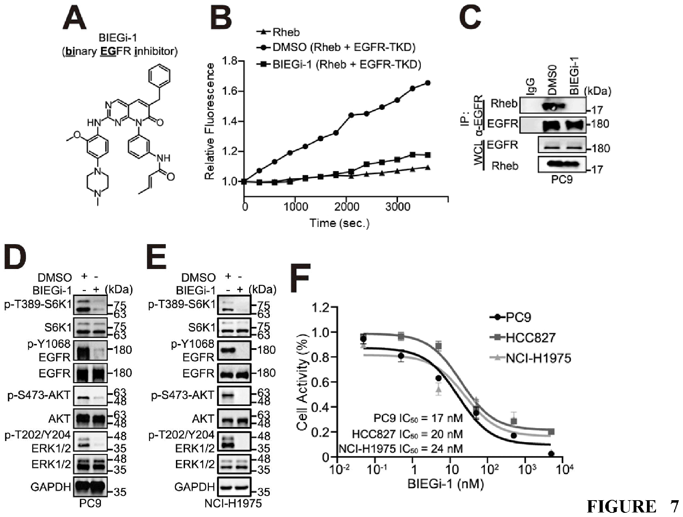

Figure 7 shows the combined inhibitory effects of the small-molecule compound BIEGi-1 on EGFR kinase and GEF functions. In Figure 7: A: Schematic representation of the chemical structure of the small-molecule compound BIEGi-1; B: BIEGi-1 inhibits EGFR-mediated Rheb nucleotide exchange; C: BIEGi-1 disrupts the interaction between EGFR and Rheb; D: In PC9 cells, BIEGi-1 inhibits the activation of mTORC1; E: In NCI-H1975 cells, BIEGi-1 inhibits the activation of mTORC1; F: BIEGi-1 inhibits EGFR-driven tumor cell proliferation.

### Detailed Description of the Invention

Through long-term and in-depth research and extensive screening, the inventors have discovered for the first time that, in addition to its conventional kinase activity, EGFR also exhibits GEF activity dependent on the glutamate residue at position 804 on its protein. This promotes Rheb (a Ras homolog) nucleotide exchange, thereby activating mTORC1 and promoting tumor growth. Furthermore, simultaneously targeting and inhibiting both the tyrosine kinase activity and GEF activity of EGFR can effectively suppress the growth of EGFR-driven tumors. Based on this, the inventors have completed the present invention.

### Therapeutic Strategy Targeting Both EGFR Kinase and GEF Activities

The inventors discovered that modulating EGFR can simultaneously regulate both EGFR kinase activity and GEF activity, and that the GEF activity of EGFR depends on its glutamate residue at position 804, which promotes Rheb nucleotide exchange, thereby activating mTORC1. Based on the above findings, the present invention further proposes a combined therapeutic strategy that simultaneously inhibits both EGFR kinase activity and GEF activity to achieve more effective treatment of tumors driven at least in part by EGFR.

The EGFR kinase activity is sustained signal transduction caused by EGFR overexpression, gene amplification, or specific mutations.

The specific mutations include, but are not limited to, the EGFRvIII variant, the L858R point mutation, or the in-frame exon-19 deletion mutation.

The present invention provides a novel strategy for treating tumors, the core of which lies in simultaneously inhibiting EGFR kinase activity and GEF activity. The tumors are those at least partially driven by EGFR, preferably including but not limited to lung cancer, colon cancer, breast cancer, endometrial cancer, thyroid cancer, glioma, squamous cell carcinoma, and prostate cancer, among others.

Specific embodiments of the novel strategy for treating tumors described in the present invention include: using one or more therapeutic agents, such as inhibitors, small-molecule compounds, nanomaterial drugs (e.g., liposomal drugs), and gene editing technologies, to achieve simultaneous targeted inhibition of EGFR kinase activity and GEF activity.

In this context, the small-molecule compounds possess the ability to penetrate cell membranes and can simultaneously interact with both the kinase domain of EGFR and the GEF activity binding site, thereby enhancing therapeutic specificity and efficacy.

In this context, the nanomaterial drugs, after surface modification, can specifically target the tumor microenvironment, and enhance affinity and selectivity for diseased tissues.

In this context, the nanomaterial drugs combine the drug with liposomes, albumin, and polymeric micelles to cross biological barriers and release drug continuously, thereby maintaining an appropriate plasma drug concentration.

In this context, the gene editing technologies (such as the CRISPR/Cas9 system) can fundamentally regulate relevant pathological processes by knocking out or modifying gene regions associated with EGFR kinase activity and GEF activity.

The present invention also provides clinical or laboratory applications of the aforementioned strategy that simultaneously targets EGFR kinase activity and GEF activity. The applications include administering one or more of the aforementioned therapeutic agents to a subject (such as a mammal or a tumor cell line) to inhibit EGFR kinase activity and GEF activity, thereby further inhibiting the mTORC1 signaling pathway, blocking tumor cell proliferation, and ultimately suppressing tumor growth.

The subjects may include humans, mice, rats, guinea pigs, pigs, dogs, monkeys, apes, etc.;
The tumor cells include lung cancer, colon cancer, and brain gliomas driven by abnormal EGFR activity.

Through a series of experiments, the inventors demonstrated that EGFR can interact directly with Rheb, and that this interaction primarily depends on the glutamate residue at position 804 of EGFR and is independent of its kinase activity.

Further experiments indicate that co-inhibition achieved by introducing the EGFR-E804K mutant via gene knock-in or by applying small-molecule compounds can effectively inhibit Rheb nucleotide exchange and mTORC1 activation, thereby suppressing tumor cell proliferation.

In summary, this invention reveals for the first time that, in addition to its traditional kinase activity, EGFR possesses a GEF function that regulates Rheb nucleotide exchange, and identifies glutamate at position 804 as a critical functional site for its GEF activity.

Based on this discovery, the present invention proposes a therapeutic strategy that simultaneously targets both the kinase and GEF activities of EGFR, providing a new theoretical basis and technical means for developing highly effective treatments for at least partial EGFR-driven tumors.

### The main Advantages of the Invention:

1. This invention expands our understanding of the molecular mechanisms of EGFR, demonstrating that in addition to its traditional tyrosine kinase activity, EGFR also possesses a GEF function that promotes the activation of the Ras homolog (Rheb). In other words, EGFR can simultaneously inhibit both its kinase and GEF activities, and the aforementioned GEF activity of EGFR depends on its glutamate residue at position 804, which promotes Rheb nucleotide exchange, thereby activating mTORC1 and ultimately promoting tumor growth.
2. The present invention provides a new therapeutic target for cancer treatment. For example, existing EGFR inhibitors primarily target the kinase domain, whereas drug-resistant tumors may rely on the GEF function of EGFR for survival. Therefore, developing inhibitors targeting the GEF domain could offer an alternative therapeutic strategy.
3. The present invention facilitates the development of novel drugs, for example, based on GEF exchange assays, a high-throughput screening platform targeting Rheb-GEF activity of EGFR can be developed to accelerate drug discovery.

The present invention will be further explained below in combination with specific examples. It should be understood that these examples are only used to illustrate the present invention and not to limit the scope of the present invention. In the following examples, the test methods without specific conditions are usually in accordance with conventional conditions or the conditions recommended by the manufacturer. Unless otherwise specified, percentages and parts are calculated by weight.

### Example 1. Preventing the activated EGFR from localizing to the lysosomal surface can significantly inhibit mTORC1 activation

### I. Experimental Methods

### 1. Cell Lysis and Lysosomal Immunoprecipitation (Lyso-IP)

Lysosomes were isolated from the non-small cell lung cancer cell line PC9 that stably expresses HA-tagged TMEM192 (a lysosomal marker protein) and from PC9 cells that stably expresses FLAG-tagged TMEM192, respectively.

First, the cells were washed with PBS, scraped, and centrifuged to collect the cell pellet. The pellet was then resuspended in KPBS buffer (composition: 136 mM KCl, 10 mM KH₂PO₄, a mixture of protease and phosphatase inhibitors, pH 7.25) and homogenized. The homogenate was centrifuged at 1,000 g for 3 minutes at 4°C, and the supernatant was collected. The supernatant was incubated with 20 µL of anti-HA magnetic beads (Beyotime) under rotation for 1 hour. Finally, the magnetic beads were washed six times with KPBS buffer, and an appropriate amount of 5 × loading buffer was added. The mixture was boiled at 100°C in a water bath for 10 minutes, cooled, and then subjected to Western blot (WB) analysis. The results are shown in Figure 1A.

### 2. Cell Lysis and Western Blotting (WB)

Non-small cell lung cancer cell lines PC9 and HCC827 harboring an EGFR exon 19 deletion mutation, and the human lung adenocarcinoma cell line NCI-H1975 harboring EGFR T790M (drug-resistant mutation) and L858R mutations were treated with the solvent DMSO, the EGFR endocytosis inhibitor Dyngo-4a, or the cathepsin C inhibitor (a late endosomal-lysosomal trafficking inhibitor) LY3000328, respectively.

The cells treated as described above were collected and lysed for 20 minutes using RIPA lysis buffer containing protease inhibitors and phosphatase inhibitors. The lysates were then centrifuged at 12,000 rpm for 20 minutes at 4°C, the supernatant was collected, and the protein concentration was quantified using the Coomassie Brilliant Blue method. The protein samples obtained were first separated by SDS-PAGE electrophoresis and then transferred to a PVDF membrane. After the membrane had been blocked, it was incubated sequentially with the primary antibody and the corresponding secondary antibody. Finally, a chromogenic substrate was added, and the expression level of the target protein was detected using an appropriate imaging system, as shown in Figure 1B.

This experiment was designed to detect the expression of the target protein in the samples and its phosphorylation levels, and the phosphorylation level of S6K1 (p-T389-S6K1) was served as an indicator of mTORC1 activation.

### II. Experimental Results

As shown in Figure 1A, Lyso-IP results indicate that the expression of both EGFR and the lysosomal marker protein LAMP1 can be detected in the PC9 cell line stably expressing HA-tagged TMEM192, suggesting that activated EGFR localizes to the lysosomal surface.

As shown in Figure 2B, compared with cells treated with DMSO, cells treated with the EGFR endocytosis inhibitor Dyngo-4a or the cathepsin C inhibitor (a late endosomal-lysosomal trafficking inhibitor) LY3000328 exhibited significantly reduced expression of phosphorylated S6K1 (p-T389- S6K1), whereas there was no difference in phosphorylated EGFR (p-Y1068-EGFR). This indicates that inhibiting EGFR endocytosis or preventing EGFR from localizing to the lysosomal surface can effectively suppress mTORC1 activation without affecting EGFR kinase activity.

Existing studies indicate that abnormally activated EGFR leads to enhanced endocytosis. In normal cells, EGFR undergoes endocytosis and enters lysosomes for degradation, thereby terminating signal transduction; however, while in tumor cells, abnormal endocytosis not only inhibits EGFR degradation but may also retain a portion of active EGFR in specific intracellular regions, thereby forming a "signaling platform" for sustained signalling that continuously and indirectly stimulates the mTORC1 signaling pathway.

The experimental results of this application indicate that inhibiting the endocytosis of EGFR or preventing EGFR from localizing to the lysosomal surface can suppress mTORC1 activation without altering EGFR kinase activity, suggesting the existence of other non-kinase-dependent regulatory mechanisms between EGFR and mTORC1.

### Example 2. EGFR and Rheb interact directly, independent of EGFR kinase activity

### I. Experimental Methods

### 1. Immunoprecipitation (IP)

### 1.1. Cell Culture and Preparation:

1.1.1. HEK293T cells were cultured in DMEM medium containing 10% fetal bovine serum and 1% penicillin-streptomycin in a humidified incubator at 37°C and 5% CO₂.

1.1.2. One day prior to transfection, HEK293T cells were seeded at an appropriate density into a 6-well cell culture plate to ensure that the cells attained appropriate confluency by the time of transfection.

### 1.2. Vector Construction

The pSIN-EGFR-WT-HA plasmid was constructed using standard PCR amplification techniques. Additionally, using the wild-type gene plasmid as a PCR template, mutation-specific primers were designed according to experimental requirements and used for amplification. PCR products were recovered by agarose gel electrophoresis, followed by ligation and transformation, and finally the success of the mutation was verified by sequencing.

### 1.3 Transient Transfection

Following the Lipofectamine 3000 protocol, the following plasmids were transfected into HEK293T cells respectively:
Vector group: Transfected with the pSIN plasmid;
WT group: Transfected with the pSIN-EGFR-WT-HA plasmid;
KD (EGFR kinase-inactive) group: following the site-directed mutagenesis method, the lysine at position 745 in the EGFR-WT-HA plasmid was mutated to alanine (K745A) to construct the EGFR-KD-HA plasmid, which was then transfected.

### 1.4 Experimental Procedures

The above cells were lysed with an appropriate amount of IP lysis buffer for 30 minutes before centrifugation, and the supernatant was collected. 20 µL of agarose beads coated with anti-HA antibody was added and incubated overnight at 4°C. Subsequently, the agarose beads-antigen-antibody complex was collected, and the supernatant was discarded. It was washed three times with pre-chilled IP elution buffer, and an appropriate amount of 5 × loading buffer was added. Then it was boiled at 100°C in a metal bath for 10 minutes, and cooled down, followed by Western blot (WB) analysis. The results are shown in Figure 2A.

### 2. Truncated Mutant Construction and IP

2.1. Using standard genetic engineering techniques, the following truncated plasmids were constructed: (as shown in Figure 2B), namely:
WT-ICD group: pSIN-EGFR-WT-ICD-V5, containing the wild-type EGFR intracellular domain;
KD-ICD group: pSIN-EGFR-KD-ICD-V5, containing the EGFR intracellular domain with the K745A mutation;
WT-TKD group: pSIN-EGFR-WT-TKD-V5, containing the wild-type EGFR intracellular tyrosine kinase domain;
KD-TKD group: pSIN-EGFR-KD-TKD-V5, containing the EGFR intracellular tyrosine kinase domain with the K745A mutation;
RD group: pSIN-EGFR-RD-V5, containing the EGFR regulatory domain but not the EGFR intracellular tyrosine kinase domain.

### 2.3 Construction of Stable Cell Lines

Using standard lentiviral infection method, a HEK293T cell line overexpressing SFB-Rheb was constructed. After successful construction, one day prior to transfection, HEK293T cells overexpressing SFB-Rheb were seeded at an appropriate density into 6-well cell culture plates to ensure that the cells reach appropriate confluency at the time of transfection.

### 2.4 Transient Transfection and IP

Following the Lipofectamine 3000 protocol, the above plasmids were transfected into the HEK293T cells overexpressing SFB-Rheb respectively. 36 hours after transfection, the above cells were lysed with an appropriate volume of IP lysis buffer for 30 minutes before centrifugation, and the supernatant was collected. 20 µL of anti-V5 agarose beads was added and incubated overnight at 4°C. Subsequently, the agarose beads-antigen-antibody complex was collected, and the supernatant was discarded. It was washed three times with pre-chilled IP elution buffer, and an appropriate volume of 5 × loading buffer was added. Then it was boiled at 100°C in a metal bath for 10 minutes, and cooled down, followed by Western blot (WB) analysis to assess the binding between EGFR and Rheb. The results are shown in Figure 2C.

### 3. Protein Purification and Pull-Down Assay

### 3.1 Protein Purification

GST and GST-Rheb proteins were induced and expressed in a prokaryotic expression system; EGFR protein was induced and expressed using an insect cell system.

The specific procedure includes: recombinant plasmids were constructed and transformed into the corresponding host cells; the cells were lysed via high-pressure lysis or sonication lysis; after centrifugation, the supernatant was separated from the cell debris; and then an affinity chromatography column was used for purification to obtain a purified protein solution.

### 3.2. Pull-down assay

The groups are as follows:
GST group: GST protein (tagged protein) purified using a prokaryotic system.
GST-Rheb group: GST-Rheb protein purified using a prokaryotic system.
EGFR-TKD-WT group: EGFR-TKD-WT protein purified using an insect cell system.

The pre-washed and equilibrated GST beads were taken, and the purified protein solutions to be tested were added as shown in the figure. The mixture was incubated at 4°C for 2 hours to ensure sufficient binding of the protein to the GST beads. After incubation, it was centrifuged at 5000 rpm for 30 seconds, and the supernatant was discarded. The beads were centrifuged and washed twice repeatedly and then collected. The beads were washed three times with pre-chilled IP elution buffer to remove non-specific bindings. An appropriate amount of loading buffer was added, then the sample was boiled at 100°C for 10 minutes to ensure complete denaturation. Subsequently, the boiled sample was stained with Coomassie Brilliant Blue to detect the target protein. The results are shown in Figure 2D.

### II. Experimental Results

Existing studies have shown that Rheb (Ras Homolog Enriched in Brain) on lysosomes is a key protein for activating mTORC1, however, whether a connection exists between EGFR and Rheb remains unknown.

As shown in Figure 2A, in HEK293T cells, both exogenously overexpressed EGFR (WT group) and kinase-deficient EGFR (KD group) can bind to endogenous Rheb, indicating that the interaction between EGFR and Rheb does not depend on EGFR kinase activity.

As shown in Figures 2B and 2C, in HEK293T cells overexpressing SFB-Rheb, the EGFR containing the EGFR intracellular tyrosine kinase domain (WT-ICD group, KD-ICD group, WT-TKD group, and KD-TKD group) were all able to interact with SFB-Rheb, whereas the EGFR containing only the EGFR regulatory domain but not the EGFR intracellular tyrosine kinase domain (RD group) could not bind, indicating that EGFR primarily binds to Rheb via its intracellular tyrosine kinase domain (TKD);
As shown in Figure 2D, in *in vitro* pull-down assays, EGFR-TKD was able to interact with GST-Rheb but not with GST, indicating that the EGFR intracellular tyrosine kinase domain (EGFR-TKD) can bind directly to Rheb.

In conclusion, these data confirm that EGFR primarily interacts directly with Rheb via the TKD, and that this process is independent of EGFR kinase activity.

### Example 3. EGFR can act as a GEF for Rheb (i.e., EGFR possesses GEF function that regulates Rheb activity)

### I. Experimental Methods: In vitro Nucleotide Exchange Assay

Rheb is a small G protein whose activity is regulated by guanine nucleotide exchange factors (GEF). Specifically, Rheb is in an activated state when bound to GTP and can activate downstream molecules, when it is hydrolyzed to GDP, it becomes inactivated, wherein GEF promote the transition of Rheb from the GDP state (inactivated state) to the GTP state (activated state), in other words, GEF facilitates Rheb nucleotide exchange.

To determine whether EGFR possesses the guanine nucleotide exchange factor (GEF) activity that regulates Rheb, the applicant used the RhoGEF Exchange Assay kit (Cytoskeleton, BK100) to conduct *in vitro* nucleotide exchange experiments, with the following groupings:
RhoA group: RhoA protein alone; RhoA is also a small G protein, and its activity is regulated by GEF;
RhoA + hDbs group: RhoA protein + hDbs protein; hDbs promotes the transition of RhoA from the GDPase (inactive) state to the GTPase (active) state, so the GEF domain of hDbs can serve as a positive control GEF;
RhoA + EGFR-TKD group: RhoA protein + EGFR-TKD protein; to determine whether EGFR possesses GEF activity that regulates RhoA;
Rheb group: Rheb protein alone;
Rheb + hDbs group: Rheb protein + hDbs protein; to determine whether hDbs possesses GEF activity that regulates Rheb;
Rheb + EGFR-TKD group: Rheb protein + EGFR-TKD protein; to determine whether EGFR possesses GEF activity that regulates Rheb.

The kit contains human RhoA protein and the GEF domains of human Dbs (hDbs), wherein, hDbs promotes the transition of RhoA from the GDPase (inactive) state to the GTPase (active) state. Therefore, GEF activity is assessed by detecting the binding of the fluorescent nucleotide analog mant-GTP to the active state of RhoA. Once mant-GTP binds to the activated GTPase, the fluorescence intensity of mant-GTP (excitation wavelength Ex: 485 ± 20 nm, emission wavelength Em: 535 ± 25 nm) will increase significantly, that is, the stronger the relative fluorescence of bound mant-GTP versus free mant-GTP, the higher the GEF activity. Therefore, this method can be used to reflect the GEF activity of the protein.

### II. Experimental Results

As shown in Figure 3, in the Rheb + EGFR-TKD group, the relative fluorescence intensity of bound mant-GTP versus free mant-GTP was similar to that in the positive control group (RhoA+hDbs group), indicating that EGFR-TKD can promote *in vitro* Rheb nucleotide exchange, i.e., EGFR-TKD possesses GEF function that regulates Rheb activity;
however, the relative fluorescence intensities of bound mant-GTP versus free mant-GTP were weaker in the RhoA+EGFR-TKD group and the Rheb+hDbs group, indicating that EGFR-TKD does not promote RhoA nucleotide exchange, nor does hDbs promote Rheb nucleotide exchange, that is, EGFR-TKD does not possess GEF function that regulates RhoA activity.

Therefore, EGFR can act as a GEF for Rheb, meaning that EGFR exhibits GEF activity.

### Example 4. Prediction of the EGFR-Rheb Binding Using AlphaFold2

### I. Experimental Methods

Structural model of the EGFR-TKD and Rheb complex was performed using AlphaFold2-multimer on Google Labs servers, subsequently, the predicted models were analyzed and presentaed using the PyMOL molecular graphics system provided by Schrödinger.

### II. Experimental Results

The pLDDT score was approximately 84.4. As shown in Figure 4, the model generated by AlphaFold2-multimer indicates that EGFR-TKD docks well with Rheb.

Based on an evaluation of the docking model, it is hypothesized that the glutamate at position 804 (E804) of EGFR may be the key amino acid site responsible for GEF activity of EGFR.

To validate this hypothesis, subsequent studies were conducted to confirm that glutamate at position 804 of EGFR is the key site for GEF activity.

### Example 5. Glutamate at position 804 of EGFR is the key site for GEF activity

### I. Experimental Methods

### 1. Construction of the EGFR-E804K Mutant

Site-directed mutagenesis was used to mutate the glutamate (E) at position 804 of EGFR to lysine (K); specific steps are described in the site-directed mutagenesis method in Example 2.

### 2. Construction of fusion protein plasmids

Different EGFR-TKD mutants (WT, KD, or E804K) were fused to the C-terminus of the LAMP2-V5 fusion protein to generate lysosome-targeted fusion proteins.

The specific method is as follows: the sequences of LAMP2 and the different EGFR-TKD variants (WT, KD, E804K) were cloned separately, and the LAMP2-V5-EGFR-TKD recombinant plasmid was constructed using the V5 tag as the linker. After all the constructions were completed, the accuracy of the sequences was confirmed via nucleotide sequencing.

### 3. IP Assay

As described in the IP method of Example 2, in brief, IP experiments were performed in HEK293T cells stably overexpressing SFB-Rheb by transiently transfecting them with the pSIN or pSIN-EGFR-WT/KD/E804K-HA plasmids shown in the figure, to detect the binding of different forms of EGFR (WT, KD, or E804K) to Rheb. The results are shown in Figure 5A.

### 4. In vitro Nucleotide Exchange Assay

The experimental method is the *in vitro* nucleotide exchange assay as described in Example 3. The RhoGEF Exchange Assay Kit (Cytoskeleton, BK100) was used to perform the *in vitro* nucleotide exchange assay to detect the ability of different forms of EGFR (WT or E804K) to promote the Rheb nucleotide exchange, i.e., GEF activity. The results are shown in Figure 5B.

### 5. Western Blot (WB) Assay

In HEK293T cells, LAMP2-V5 or LAM2-V5-EGFR-TKD-WT/KD/E804K was stably overexpressed. After the successful expression was conformed, the cells were serum-starved for 24 hours to assess mTORC1 activation. The results are shown in Figure 5C.

### II. Experimental Results

As shown in Figure 5A, compared with the EGFR-WT and EGFR-KD groups, no protein bands were detected in the EGFR-E804K group, indicating that EGFR with a glutamate mutation at position 804 cannot interact with Rheb. Thus, the glutamate at position 804 of EGFR (E804) is a key site for the interaction between EGFR and Rheb.

As shown in Figure 5B, compared with the Rheb+EGFR-TKD-WT group, the EGFR-TKD-E804K group exhibited weaker relative fluorescence intensity, indicating that EGFR harboring a mutation at glutamate 804 cannot promote Rheb nucleotide exchange *in vitro.* In other words, the glutamate at position 804 (E804) of EGFR is a key site for GEF activity of EGFR.

As shown in Figure 5C, LAMP2-V5-EGFR-TKD-WT and LAMP2-V5-EGFR-TKD-KD both effectively activated mTORC1 after being targeted to the lysosomal surface, whereas LAMP2-V5-EGFR-TKD-E804K failed to activate mTORC1 after being targeted to the lysosomal surface, further indicating that the glutamate at position 804 (E804) of EGFR is a key site for EGFR to regulate mTORC1 .

The above results clearly demonstrate that the glutamate at position 804 (E804) of EGFR is a key site for its GEF activity and, consequently, for regulating the activation of mTORC1.

### Example 6. PC9 cells with endogenous EGFR-E804K knock-in fail to activate mTORC1 and inhibit tumor growth

### I. Experimental Methods

### 1. Construction of PC9 cells with endogenous EGFR-E804K knock-in

sgRNAs targeting EGFR were designed and screened for high-efficacy sequences, while a donor DNA template containing the E804K mutation was constructed. The donor DNA template was constructed into the pSIN vector (without the EF1α promoter) and integrated with a gene encoding EGFP and an upstream internal ribosomal entry site (IRES) to enhance the screening efficiency of positive clones.

The sgRNA and donor DNA template were co-transfected into parental PC9 cells. Approximately 3 days post-transfection, Alexa Fluor-488-positive single cells were sorted using a MoFloAstrios EQ cell sorter (Beckman Coulter), and positive clones were validated via PCR and Sanger sequencing to confirm the successful construction of PC9 cells with endogenous EGFR-E804K knock-in.

### 2. Tumor Cell Proliferation Assay

The proliferation capacity of parental PC9 cells (Parent group) and EGFR-E804K gene-knock-in PC9 cells (E804K group) was evaluated using the CCK-8 assay, colony formation assay, and the EdU (Beyotime, C0071) assay.

### 3. Subcutaneous Xenograft Tumor In Nude Mice

The animal experiments of this invention have been approved by the Animal Research Committee of the Cancer Center of Sun Yat-sen University (Approval No. L025501202410005).

Four-week-old female BALB/c nude mice weighing 18-20 g were purchased from Zhuhai Best Biotechnology Co., Ltd. (Zhuhai, China).

Mice were randomly assigned to two groups, with six mice in each group. To assess tumor growth, 5 × 10⁶ parental PC9 cells or EGFR-E804K knock-in PC9 cells were subcutaneously injected into the axillae of the mice, constituting the parental group and the E804K group, respectively. Starting from one week after injection, tumor volume was measured every three days using a vernier caliper, the volume was calculated using the formula: V (volume) = (width² × length × π) / 6. After 5 weeks of growth, the mice were euthanized with 100% CO₂ at a flow rate of 30-70% of chamber volume per minute. The tumors were collected, and the volumes and weights were measured using a vernier caliper and an electronic balance, respectively. The results are shown in Figures 6E, 6F, and 6G.

### II. Experimental Results

As shown in Figure 6A, compared with parental PC9 cells (parental group), there was no difference in phosphorylated EGFR (p-Y1068-EGFR) in EGFR-E804K knock-in PC9 cells (E804K group), however, the expression of phosphorylated S6K1 (p-T389-S6K1) was significantly reduced. This indicates that although EGFR-E804K-knock in PC9 cells (E804K group) can retain normal EGFR kinase activity, they are unable to activate mTORC1, further demonstrating that glutamate at position 804 (E804) of EGFR is a key site for EGFR to regulate mTORC1.

As shown in Figures 6B (CCK-8 assay), 6C (colony formation assay), and 6D (EdU assay), compared with parental PC9 cells (Parental group), EGFR-E804K knock-in PC9 cells (E804K group) exhibited significantly reduced proliferative capacity, indicating that inhibition of glutamate at position 804 of EGFR (E804) (that is, inactivating EGFR) can effectively inhibit tumor growth;

as shown in Figures 6E, 6F, and 6G, compared with the parental group subcutaneously inoculated with parental PC9 cells, the tumor volume and weight were significantly decreased in the nude mouse subcutaneous xenograft model injected with EGFR E804K knock-in PC9 cells (E804K group), further demonstrating that inhibition of glutamate at position 804 of EGFR (E804) can effectively suppress tumor growth.

In summary, PC9 cells with endogenous EGFR-E804K knock-in (i.e., EGFR-inactivated PC9 cells) are unable to activate mTORC1, thereby inhibiting tumor growth.

### Example 7. Combined inhibition of small-molecule compound BIEGi-1 on EGFR kinase and GEF functions

### I. Experimental Methods

### 1. Screening and Identification of Small-Molecule Compounds That Inhibit EGFR Kinase Activity

Studies have reported that 7-oxopyridopyrimidine compounds represent a novel class of EGFR tyrosine kinase inhibitors with the potential to overcome the issues of poor selectivity towards wild-type cells and toxic side effects associated with traditional EGFR inhibitors.

As described in the *in vitro* nucleotide exchange experiment in Example 3, the aforementioned 7-oxopyridopyrimidine compounds were screened using the RhoGEF Exchange Assay Kit (Cytoskeleton, BK100). The results are shown in Figures 7A and 7B.

The groups were defined as follows:
Rheb group: Rheb protein alone.
DMSO (Rheb+EGFR-TKD) group: Rheb+EGFR-TKD with DMSO added, serving as the control group.
BIEGi-1 (Rheb+EGFR-TKD) group: Rheb+EGFR-TKD with BIEGi-1 added, i.e., the treatment group.

### 2. IP Experiment

As described in the IP method in Example 2. Immunoprecipitation experiments were performed in PC9 cells. Cells from the control and treatment groups were lysed with an appropriate volume of IP lysis buffer for 30 minutes before centrifugation, and the supernatant was collected. 1 µg of the corresponding antibody and 50 µL of Protein A/G beads were added, and the mixture was incubated overnight at 4°C. Subsequently, the agarose beads-antigen-antibody complex was collected, and the supernatant was discarded, and the complex was washed three times with pre-chilled IP elution buffer. After boiled at 100°C for 10 minutes, the sample was cooled and subjected to Western blot (WB) analysis to determine whether BIEGi-1 could inhibit the binding of EGFR to Rheb. The results are shown in Figure 7C.

### 3. Drug Treatment Experiments.

PC9 and NCI-H1975 cells were treated with DMSO or BIEGi-1, respectively, and the activation of mTORC1 was assessed. The results are shown in Figures 7D and 7E.

### 4. IC₅₀ Determination Method

An appopriate number of target cells was seeded into a 96-well plate and cultured at 37°C and 5% CO₂ for 24 hours; a series of drug solutions to be tested at different concentrations was prepared, with at least three replicate wells for each concentration, and a control group treated with an equal volume of culture medium was set up; after 72 hours of drug treatment, 10 µL of CCK-8 reagent was added to each well and incubated in the incubator for 1 hour; the optical density (OD) of each well at 450 nm was measured using a microplate reader; the data was processed using GraphPad Prism 9.0 software to calculate the cell survival rate for each concentration (formula: Survival rate = [(Treated group OD - Blank OD)/(Control group OD - Blank OD)] × 100%); nonlinear regression analysis was performed to determine the IC₅₀ value, i.e., the drug concentration required to inhibit cell proliferation by 50%. The results are shown in Figure 7F.

### II. Experimental Results

As shown in Figure 7B, compared with DMSO, the addition of the small-molecule compound (BIEGi-1) shown in Figure 7A resulted in weaker relative fluorescence of bound mant-GTP and free mant-GTP, indicating that BIEGi-1 can significantly inhibit the nucleotide exchange capacity of Rheb mediated by EGFR, in other words, BIEGi-1 can inhibit the GEF activity of EGFR.

As shown in Figure 7C, compared with the DMSO group, the protein expression of Rheb was significantly reduced in the BIEGi-1 group, indicating that BIEGi-1 can disrupt the interaction between EGFR and Rheb .

As shown in Figure 7D, in PC9 cells, compared with the DMSO group, the protein expression of phosphorylated S6K1 (p-T389-S6K1) was significantly reduced in the BIEGi-1 group, indicating that BIEGi-1 can inhibit mTORC1 activation.

As shown in Figure 7F, in NCI-H1975 cells, compared with the DMSO group, the protein expression of phosphorylated S6K1 (p-T389-S6K1) was significantly reduced in the BIEGi-1 group, indicating that non-small cell lung cancer cell lines harboring the EGFR-T790M resistance mutation (the T790M mutation is one of the key mechanisms underlying resistance to EGFR kinase inhibitors) remain sensitive to BIEGi-1, that is, BIEGi-1 can inhibit mTORC1 activation in cell lines resistant to EGFR kinase inhibitors.

As shown in Figure 7E, in the non-small cell lung cancer cell lines PC9, HCC827, and NCI-H1975 harboring EGFR-mutations, the IC₅₀ values of BIEGi-1 were 17 nM, 20 nM, and 24 nM, respectively, indicating that BIEGi-1 possesses an excellent ability to inhibit EGFR-driven tumor cell proliferation.

In summary, in addition to its known role in regulating EGFR tyrosine kinase activity, EGFR also possesses a GEF function that promotes Rheb nucleotide exchange, which primarily depends on the glutamate residue at position 804 of EGFR, thereby further linking EGFR to the mTORC1 signaling pathway.

Based on the above findings, the combined targeted inhibition of both the kinase and GEF activities of EGFR using inhibitors, small-molecule compounds, nanomaterials, or gene-editing technologies could serve as a new strategy for treating tumors driven at least in part by EGFR, provide new technical insights and methods for treating tumors driven mainly by EGFR, and also lay a solid experimental foundation for the development of highly effective antitumor drugs. This strategy exhibits significant application prospects and important clinical significance.

All documents mentioned herein are incorporated by reference in this invention as if each document was individually incorporated by reference. In addition, it should be understood that after reading the above teaching content of the present invention, those skilled in the art can make various changes or modifications to the present invention, and these equivalent forms also fall within the scope defined by the appended claims of the present invention.

## Claims

1. A use of an EGFR inhibitor, wherein, it is used for the preparation of a pharmaceutical composition for treating diseases mediated by guanine nucleotide exchange factor (GEF) activity, or co-mediated by guanine nucleotide exchange factor (GEF) activity and EGFR tyrosine kinase activity.

2. The use according to claim 1, wherein, the disease is a cancer selected from the group consisting of: lung cancer, colorectal cancer, breast cancer, endometrial cancer, thyroid cancer, glioma, squamous cell carcinoma, prostate cancer, melanoma, leukemia, and glioblastoma.

3. The use according to claim 1, wherein, the pharmaceutical composition is further used to modulate the abnormal Rheb nucleotide exchange; preferably, the regulation is independent of the regulation of kinase activity or expression abnormalities of EGFR.

4. The use according to claim 1, wherein, the pharmaceutical composition simultaneously modulates EGFR tyrosine kinase activity and GEF activity, and the regulation of GEF activity is independent of the regulation of EGFR tyrosine kinase activity.

5. The use according to claim 1, wherein, the EGFR inhibitor is selected from the group consisting of: small-molecule compounds, nanomaterial drugs, gene editing technologies, protein antibodies, nucleic acid drugs, enzyme drugs, expression vectors, cell therapies, hormone drugs, peptide drugs, or combinations thereof.

6. The use according to claim 5, wherein, the EGFR inhibitor is a small-molecule compound.

7. The use according to claim 6, wherein, the EGFR inhibitor is a small-molecule compound as shown in the following formula:

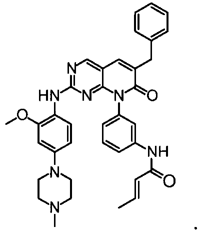

8. A pharmaceutical combination, wherein, it comprises an EGFR tyrosine kinase inhibitor and a GEF activity inhibitor, or a dual-target inhibitor of EGFR tyrosine kinase/GEF activity.

9. A use of the pharmaceutical combination according to claim 8, wherein, it is used for the preparation of a pharmaceutical composition for treating tumors driven by EGFR or resistant to a single EGFR tyrosine kinase inhibitor;
preferably, the tumor is selected from the group consisting of: lung cancer, colorectal cancer, breast cancer, endometrial cancer, thyroid cancer, glioma, squamous cell carcinoma, prostate cancer, melanoma, leukemia, and glioblastoma.

10. A method for designing a dual-target inhibitor of EGFR tyrosine kinase and GEF activity, wherein, it comprises the steps of:
(a) providing a candidate molecule, and the candidate molecule can simultaneously act on the kinase domain of EGFR and the glutamate residue at position 804 of EGFR;
(b) determining the inhibitory ability of the candidate molecule against EGFR kinase and GEF activity.
